# FASCICULE DE BREVET EUROPEEN

(11) **EP 1 161 251 B1**
(45) Date de publication et mention de la délivrance du brevet: **28.05.2003**
(21) Numéro de dépôt: 00910979.4
(22) Date de dépôt: 17.03.2000
(51) Int. Cl.: A61K 35/74, A61K 7/26, A61P 1/02

(54) **UTILISATION D'UNE COMPOSITION BACTERIENNE COMPRENANT DES PROTEOGLYCANES DE KLEBSIELLA PNEUMONIAE ET DES RIBOSOMES OU DES ARN RIBOSOMAUX BACTERIENS, POUR LA PREPARATION D'UN MEDICAMENT POUR LE TRAITEMENT DES MALADIES PARODONTALES**
VERWENDUNG EINER ZUSAMMENSETZUNG ENTHALTEND PROTEOGLYCANE VON KLEBSIELLA PNEUMONIAE UND BAKTERIELLE RIBOSOME ODER RIBOSOMALE RNS, ZUR HERSTELLUNG EINES MEDIKAMENTES ZUR BEHANDLUNG PARADONTALER ERKRANKUNGEN
USE OF A COMPOSITION COMPRISING KLEBSIELLA PNEUMONIAE PROTEOGLYCANS, AND BACTERIAL RIBOSOMES OR RIBOSOMAL RNA'S FOR THE PREPARATION OF A MEDICAMENT FOR TREATING AND/OR PREVENTING PERIODONTAL DISEASES

(30) Priorité: 17.03.1999 FR 9903306
(43) Date de publication de la demande: 12.12.2001
(73) Titulaire: PIERRE FABRE SANTE, 92100 Boulogne-Billancourt (FR)
(72) Inventeur: LIBON, Christine, F-74160 Saint Julien en Genevois (FR); CORVAIA, Nathalie, F-74160 St. Julien en Genevois (FR); FEDERLIN, Ducani, Marie, F-81100 Castres (FR)
(74) Mandataire: Warcoin, Jacques
(86) Numéro de dépôt international: FR0000659
(87) Numéro de publication internationale: WO00054728

(56) Documents cités:
- EP-A- 0 013 851
- EP-A- 0 238 407
- FR-A- 2 388 563
- US-A- 3 931 398

## Description

La présente invention a pour objet l'utilisation d'une composition comprenant des protéoglycanes de Klebsiella pneumoniae, et des ribosomes ou des ARNs ribosomaux de Klebsiella pneumoniae, de Streptococcus pneumoniae, de Streptococcus pyogenes et d'Haemophilus influenzae, notamment le D53, pour la préparation d'un médicament destiné au traitement et/ou à la prévention des maladies parodontales ou de leur récidive, ou de la plaque dentaire.

L'agent étiologique principal incriminé dans la succession des situations inflammatoires et immunitaires des maladies parodontales est la composante bactérienne de la plaque dentaire. Les parodontopathies constituent un ensemble d'infections polymicrobiennes de type mixte, mais à prédominance anaérobie. A chaque manifestation clinique d'une parodontopathie, semble être corrélé un groupe spécifique de bactéries qui s'y distinguent par leur incidence élevée, avec en particulier les bactéries à Gram négatif parmi lesquelles on distingue en particulier (Periodontology 2000, 1994, 5 : 78-111) :
- Actinobacillus actinomycetemcomitans
- Fusobacterium nucleatum
- Porphyromonas gingivalis
- Prevotella intermedia.

Parallèlement à cet état de faits, il existe des facteurs, soit locaux, soit systémiques, qui peuvent moduler la pathogénèse de la maladie parodontale. Les facteurs systémiques jouent un rôle prépondérant dans l'incidence et la progression des parodontites. En effet, tout facteur capable d'altérer la réponse inflammatoire / immunitaire et ainsi l'équilibre hôte-bactéries, aura un effet sur la santé parodontale.

Ainsi, partant du postulat que la maladie parodontale est une maladie de site, toute modification de l'immunité locale au niveau de ce ou de ces sites a une incidence directe sur le développement et la progression de la pathologie.

A l'heure actuelle, le traitement des maladies parodontales réside essentiellement en l'instauration d'un contrôle de plaque rigoureux visant à éliminer l'agent causal, en l'occurrence la plaque bactérienne. Ce traitement peut être chirurgical ou non chirurgical et doit conduire à la stabilisation, voire la guérison, des lésions.

Dans le cas où le plan de traitement préconisé aboutit à un échec, bien que l'agent étiologique ait été éliminé, la persistance de la pathologie est vraisemblablement en relation avec une altération des mécanismes responsables de la réponse de l'hôte, et plus particulièrement de l'immunité locale.

Des composés ont été étudiés pour le traitement ou la prévention d'infection responsable de parodontopathie. En général, ces composés, capables de réduire ou d'éliminer le pouvoir infectieux de ces micro-organismes, sont soit constitués d'agent bactéricide ou de lectine, tel que décrit par exemple dans EP 0 819 380 ou WO 96/24368, ou d'agent immunogène constitué d'antigène spécifique de ces micro-organismes.

De nombreux candidats vaccins composés de ribosomes ou d'ARNs ribosomaux et de protéoglycanes de bactéries à Gram négatifs ont déjà été décrits comme adjuvant de l'immunité ou comme agent immunogène pour le traitement prophylactique d'infection (EP 0 238 407, EP 0 035 429, FR 78 35 649 ou FR 73 43 957). Ces documents mentionnent que les souches dont sont issues les fractions ribosomales constituant ces vaccins correspondent aux souches responsables de l'infection à traiter, ces ribosomes ou ARNs ribosomaux constituant la fraction antigénique de ces vaccins.

Parmi ces documents, on peut citer le document FR 78 35 649 qui décrit une composition vaccinale comprenant des protéoglycanes de Klebsiella pneumoniae, et des ARNS ribosomaux de Klebsiella pneumoniae, de Streptococcus pneumoniae, de Streptococcus pyogenes et d'Haemophilus influenzae, telle que le D53, pour le traitement prophylactique des infections bronchiques et de la sphère ORL.

On peut citer en outre les travaux de L. Hbabi-Haddioui et al. (Drugs, 1997, 54, Suppl. 1, 29-32) décrivant l'inhibition de l'adhésion de Streptococcus pneumoniae sur l'épithélium buccal par des IgA salivaires spécifiques des souches dont sont issus les ribosomes constituant la composition vaccinale utilisée pour l'immunisation.

On peut citer également un candidat vaccin contre les parodontopathies décrit dans le brevet EP 0 238 407 constitué de ribosomes d'Actinomyces viscosus, d'Actinomyces naeslundii et de Veillonella parvula, et de protéoglycanes de Klebsiella pneumoniae.

Ainsi, il existe aujourd'hui un besoin de disposer de composition de type vaccinal permettant de renforcer l'immunité de l'hôte contre les micro-organismes responsables des maladies parodontales, en particulier contre les bactéries à Gram négatif d'incidence élevée associées à ces maladies.

De manière surprenante, il a été mis en évidence qu'une immunisation réalisée avec une composition comprenant des protéoglycanes de Klebsiella pneumoniae, et des ARNS ribosomaux de Klebsiella pneumoniae, de Streptococcus pneumoniae, de Streptococcus pyogenes et d'Haemophilus influenzae, telle que le D53, permet de générer chez l'hôte des anticorps capables de reconnaître des antigènes de souches bactériennes d'incidence élevée dans les maladies parodontales et qu'ainsi une telle composition pouvait être utilisée en tant que vaccin pour le traitement et/ou la prévention des maladies parodontales ou de leur récidive.

La présente invention a ainsi pour objet l'utilisation d'une composition comprenant des protéoglycanes de Klebsiella pneumoniae, et des ribosomes ou des ARNS ribosomaux de Klebsiella pneumoniae, de Streptococcus pneumoniae, de Streptococcus pyogenes et d'Haemophilus influenzae pour la préparation d'un médicament destiné au traitement et/ou à la prévention des maladies parodontales ou de leur récidive.

De préférence, les protéoglycanes de Klebsiella pneumoniae de ladite composition selon l'invention sont solubles dans l'eau, purifiés et apyrogènes. Lesdits protéoglycanes de Klebsiella pneumoniae pourront par exemple être obtenus par des procédés tels que ceux décrits aux exemples 1 à 4 du brevet FR 78 35 649, ou ceux décrits dans les exemples 2 et 3 du brevet EP 0 238 407, ou par un procédé équivalent.

De préférence également, les ribosomes ou ARNs ribosomaux de Klebsiella pneumoniae, de Streptococcus pneumoniae, de Streptococcus pyogenes et d'Haemophilus influenzae de ladite composition selon l'invention pourront être obtenus par des procédés connus tels que ceux décrits par exemple dans les brevets FR 73 43 957, FR 75 10 252, FR 76 24 124, ou ceux décrits dans les exemples 4 à 7 du brevet EP 0 238 407, ou par un procédé équivalent.

L'invention concerne plus particulièrement l'utilisation selon l'invention, caractérisée en ce que ladite composition comprend des protéoglycanes membranaires purifiés de Klebsiella pneumoniae et des ribosomes de Klebsiella pneumoniae, de Streptococcus pneumoniae, de Streptococcus pyogenes et d'Haemophilus influenzae.

De manière préférée, lesdits ribosomes de Klebsiella pneumoniae, de Streptococcus pneumoniae, de Streptococcus pyogenes et d'Haemophilus influenzae selon l'invention contiennent plus de 65 % (p/p), de préférence 70 % (p/p) d'ARNs.

De manière également préférée, le rapport en poids entre lesdits protéoglycanes et lesdits ribosomes ou ARNs ribosomaux de la composition selon l'invention est compris entre 1,4 et 1,6, de préférence 1,5.

Dans un mode de réalisation particulier, l'invention comprend l'utilisation selon l'invention, caractérisée en ce que les ribosomes ou ARNs ribosomaux de Klebsiella pneumoniae, de Streptococcus pneumoniae, de Streptococcus pyogenes et d'Haemophilus influenzae sont contenus dans ladite composition à un taux respectif compris entre 25 % et 45 %, 20% et 40 %, 20 % et 40 %, et 1% et 10 % en poids ou partie par rapport à la quantité totale de ribosomes ou d'ARNs ribosomaux de Klebsiella pneumoniae, de Streptococcus pneumoniae, de Streptococcus pyogenes et d'Haemophilus influenzae, de préférence à un taux respectif de 35 %, 30 %, 30% et 5 %.

L'invention comprend en outre l'utilisation d'une composition selon l'invention, caractérisée en ce que ladite composition comprend en outre un excipient pharmaceutiquement acceptable choisi parmi ceux bien connus pour la formulation de composition pharmaceutique, notamment de type vaccinal. Ces excipients seront en particulier choisis en fonction de la présentation de la composition (solution, poudre etc.) ou du mode d'administration de la composition, tels que notamment les glycérides polyglycosylés insaturés, l'eucalyptol, le dichlorodifluorométhane, le polyvidone et le mannitol.

L'invention comprend en outre l'utilisation selon l'invention, caractérisée en ce que ledit médicament est administré par voie nasale, orale, parentérale ou par voie topique.

Dans un mode de réalisation préféré, l'invention a pour objet l'utilisation d'une composition pour la préparation d'un médicament destiné au traitement et/ou à la prévention des maladies parodontales selon l'invention, caractérisée en ce que ledit médicament est présenté sous forme :
1) d'une suspension pour inhalation de composition suivante :
   pour un flacon pressurisé de 10 ml
   - fractions ribosomales :
      ribosomes titrés à 70 % d'ARN 2 mg
      associés dans les proportions suivantes :
      ribosomes de Klebsiella pneumoniae 35 parties
      ribosomes de Streptococcus pneumoniae 30 parties
      ribosomes de Streptococcus pyogenes groupe A 30 parties
      ribosomes d'Haemophilus influenzae 5 parties
   - fractions membranaires :
      protéoglycanes de Klebsiella pneumoniae 3 mg
   - excipients :
      glycérides polyglycosylés insaturés, eucalyptol, dichlorodifluorométhane ;
2) d'un sachet de granulé pour solution buvable de composition suivante :
   - fractions ribosomales :
      ribosomes titrés à 70 % d'ARN 0,750 mg
      associés dans les proportions suivantes :
      ribosomes de Klebsiella pneumoniae 35 parties
      ribosomes de Streptococcus pneumoniae 30 parties
      ribosomes de Streptococcus pyogenes groupe A 30 parties
      ribosomes d'Haemophilus influenzae 5 parties
   - fractions membranaires :
      protéoglycanes de Klebsiella pneumoniae 1,125 mg
   - excipients :
      polyvidone, D-mannitol ; ou
3) d'une poudre lyophilisée à réhydrater pour usage parentéral de composition suivante :
   - lyophilisât : par flacon
      - fractions ribosomales :
         ribosomes titrés à 70 % d'ARN 0,010 mg associés dans les proportions suivantes :
         ribosomes de Klebsiella pneumoniae 35 parties
         ribosomes de Streptococcus pneumoniae 30 parties
         ribosomes de Streptococcus pyogenes groupe A 30 parties
         ribosomes d'Haemophilus influenzae 5 parties
      - fractions membranaires :
         protéoglycanes de Klebsiella pneumoniae 0,015 mg
      - excipient :
         mannitol
   - solvant : par ampoule
      chlorure de sodium 3,5 mg
      eau pour préparations injectables 0,5 ml.

L'invention comprend enfin l'utilisation d'une composition comprenant des protéoglycanes de Klebsiella pneumoniae, et des ribosomes ou des ARNs ribosomaux de Klebsiella pneumoniae, de Streptococcus pneumoniae, de Streptococcus pyogenes et d'Haemophilus influenzae, pour la préparation d'une composition cosmétique destinée au traitement et/ou à la prévention de la plaque dentaire qui peut entraîner plus ou moins rapidement un effet inesthétique.

Bien entendu, ladite composition cosmétique selon l'invention, comprendra en particulier les compositions de protéoglycanes de Klebsiella pneumoniae, et de ribosomes ou d'ARN ribosomaux de Klebsiella pneumoniae, de Streptococcus pneumoniae, de Streptococcus pyogenes et d'Haemophilus influenzae telles que définies préalablement dans la présente description, sous une forme identique, ou sous une forme, quantité, dosage et/ou avec des additifs particulièrement adaptés à son utilisation cosmétique.

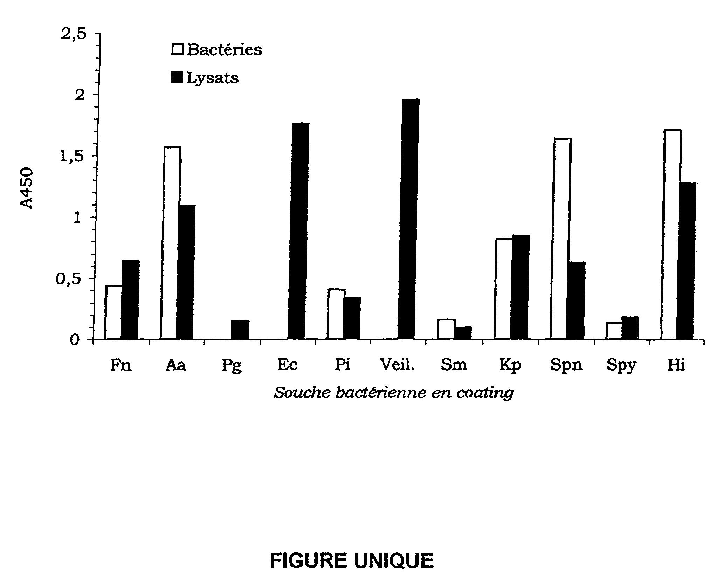

Les exemples ci-après, ainsi que la légende de la figure unique ci-dessous illustrent la présente invention ainsi que d'autres avantages ou caractéristiques de l'invention.

### Légende de la figure

Réactivité d'un sérum de souris anti-J022X (lot co115) vis-à-vis de différentes souches bactériennes.

La figure représente la valeur de l'absorption obtenue à 450 nm par ELISA (A450) pour un sérum de souris anti- co115 par rapport à un sérum témoin (A450 = (A450 sérum anti-col15) - (A450 sérum irrelevant), dilution du sérum au 1:1600) pour chaque antigène de capture déposé initialement sur la plaque de microtitration.
Fn : Fusobacterium nucleatum ; Aa : Actinobacillus actinomycetemcomitans ; Pg : Porphyromonas gingivalis ; Ec : Eikenella corrodens ; Pi : Prevetella intermedia ; Veil : Veillonella spp ; Sm : Streptococcus mutans ; Kp : Klebsiella pneumoniae ; Spn : Streptococcus pneumoniae ; Spy : Streptococcus pyogenes ; Hi : Haemophilus influenzae ; n.t. : non testé.
□ : Antigène de capture sous forme de bactéries entières ;
■ : Antigène de capture sous forme de lysats bactériens.

### EXEMPLES

### Exemple 1 : réactifs utilisés

### Composition utilisée pour l'immunisation : D53 ( J022X lot col15)

- fractions ribosomales :
   ribosomes titrés à 70 % d'ARN 0,010 mg
   associés dans les proportions suivantes :
   ribosomes de Klebsiella pneumoniae 35 parties
   ribosomes de Streptococcus pneumoniae 30 parties
   ribosomes de Streptococcus pyogenes groupe A 30 parties
   ribosomes d'Haemophilus influenzae 5 parties
- fractions membranaires :
   protéoglycanes de Klebsiella pneumoniae : 0,015 mg
- excipient : mannitol
   chlorure de sodium : 3,5 mg
   eau pour préparations injectables : 0,5 ml.

### Souches bactériennes

- Klebsiella pneumoniae : Kp I145 souche D53
- Streptococcus pneumoniae : Spn souche D53
- Streptococcus pyogenes : Spv souche D53
- Haemophilus influenzae : Hi souche D53
- Fusobacterium nucleatum : Fn CIP 101130T
- Actinobacillus actinomycetemcomitans : Aa CIP 52106T
- Porphyromonas gingivalis : Pg AIP 103683 (ATCC 33277)
- Eikenella corrodens : Ec CIP 7075T
- Prevetella intermedia : Pi
- Veillonella spp : Veil. CIP 4468
- Streptococcus mutans : Sm CIP 103 220T.

### Préparation des bactéries entières utilisées comme antigène de capture dans le premier test ELISA

- Les souches sont repiquées sur milieu solide Colombia à 5 % de sang de mouton. Les boîtes sont mises à incuber à 37°C sous anaérobiose. Le repiquage se fait tous les 3 à 4 jours.
- Préparation d'une suspension de bactéries.

Les colonies bactériennes sont écouvillonnées directement sur les boîtes de culture et remises en suspension. La densité optique est mesurée à λ = 640 nm afin d'ajuster la suspension à 10⁸ bactéries/ml.

### Préparation des lysats bactériens utilisés comme antigène de capture dans le second test ELISA

- Préparation d'une suspension de bactéries en PBS (tampon phosphate salin) :
   - écouvillonnage de 6 ou 7 géloses au sang ensemencées par inondation, mises en suspension dans du PBS,
   - lavage par centrifugation de la suspension (10 min à 3 500 g) et reprise dans environ 5 ml de PBS,
   - congélation à - 20°C.
- Lyse bactérienne :
   - décongélation de la suspension bactérienne,
   - addition à la suspension de bactéries d'1 ml de réactif Qiagen (composition : SDS 1 % + NaOH 200 mM),
   - agitation par retournement du mélange,
   - temps de lyse : 3 à 5 min à température ambiante,
   - contrôle microbiologique (comme indiqué ci-dessous) et dosage de protéines par BCA,
   - congélation à - 20°C.
- Contrôle microbiologique de la lyse :
   - avec 0,1 ml de lysat, ensemencement par inondation d'une gélose et incubation dans les conditions de culture de la souche. Pour que la lyse soit considérée comme totale, il faut observer une absence de développement microbien (soit < 10 germes / ml.).

### Sérums utilisés pour l'ELISA

- sérum de souris OF1 naïves,
- sérum témoin irrelevant : sérum de souris BALB/c immunisées avec un conjugué KLH-peptide de virus de la rougeole,
- sérum anti-col15 : sérum prélevé au bout de 21 jours après immunisation de souris OF1 immunisées par voie intra-péritonéal (i.p.) au jour J0, J7 et J14 avec *250* µg de D53 J022X lot col15 (correspondant à 100 µg de fractions ribosomales et à 150 µg de fractions membranaires).

### Protocole des deux tests ELISA utilisés

a) Premier test ELISA utilisant des bactéries entières comme antigène de capture Sur plaques Immulon II (Dynatech, Chantilly, Virginia, USA) :
   - dépôt de 100 µl/puits d'une suspension bactérienne à 10⁸ bactéries/ml en tampon carbonate/bicarbonate pH 9,8. Incubation toute la nuit à + 4°C ;
   - lavages en tampon PBS (tampon phosphate salin), tween 0,05 % ;
   - saturation avec de l'albumine bovine fraction V à 3 % (p/v) en PBS pendant 2 h à 37°C ;
   - lavages en tampon PBS, tween à 0,05 % ;
   - dépôt de 100 µl/puits de dilutions de raison 2 des différents sérums dilués dans une solution de Tween 0,05 % et d'albumine à 0,5 % en tampon PBS puis incubation 2 h à 37°C ;
   - lavages en tampon PBS, tween à 0,05 % ;
   - dépôt de 100 µl/puits d'anticorps secondaires de chèvre anti-souris couplés à la péroxydase, dilué au 1:5000 (goat anti-mouse IgG, Pierce réf 31438, Rockford, USA) ;
   - incubation 1 h à 37°C ;
   - lavages en tampon PBS, tween à 0,05 % ;
   - ajout de 100 µl/puits de TMB (3,3',5,5' tétraméthylbenzidine), substrat de la péroxydase, Microwell (Pierce, Rockford, USA) et incubation pendant 10 minutes ;
   - ajout de 100 µl/puits d'acide sulfurique 1M pour arrêter la réaction puis lecture de l'absorbance de la solution obtenue à une longueur d'onde de 450 nm.
b) Second test ELISA utilisant des lysats bactériens comme antigène de capture
   Sur plaques Immulon II (Dynatech, Chantilly, Virginia, USA) :
   - dépôt de 100 µl/puits de lysat bactérien à 10 µg de protéines/ml en tampon carbonate/bicarbonate pH 9,8. Incubation toute la nuit à + 4°C ;
   - lavages en tampon PBS (tampon phosphate salin) (Seromed, Berlin, Germany) ;
   - saturation avec de la gélatine à 0,5 % (p/v) en PBS (Serva, Heidelberg, Germany) pendant 1 h à 37°C ;
   - lavages en tampon PBS ;
   - dépôt de 100 µl/puits de dilutions de raison 2 des différents sérums dilués dans une solution de Tween 0,05 % et de gélatine 0,1 % en tampon PBS puis incubation 2 h à 37°C ;
   - lavages en tampon PBS ;
   - dépôt de 100 µl/puits d'anticorps secondaires de chèvre anti-souris couplés à la péroxydase, dilué au 1:5000 (goat anti-mouse IgG, Pierce réf 31438, Rockford, USA) ;
   - incubation 1 h à 37°C ;
   - lavages en tampon PBS ;
   - ajout de 100 µl/puits de TMB, substrat de la péroxydase, Microwell (Pierce, Rockford, USA) et incubation pendant 10 minutes ;
   - ajout de 100 µl/puits d'acide sulfurique 1M pour arrêter la réaction puis lecture de l'absorbance de la solution obtenue à une longueur d'onde de 450 nm.

### Contrôles :

Sérum de souris naïves : sérum de souris non immunisées.
Blanc réactif pour vérifier la non interférence des différents réactifs, hors sérum, utilisés dans le test ELISA.

### Seuil de détection :

Déterminé par le sérum témoin irrelevant, c'est-à-dire riche en anticorps et n'ayant aucune communauté antigénique avec l'antigène étudié. Ce seuil permet d'estimer les réactions non spécifiques dans le sérum testé.

### Exemple 2 : Réactions croisées entre souches bactériennes de D53 et souches parodontales

Le but de cette étude est de savoir si un sérum de souris immunisées avec J022X (lot col15), principe actif de D53 induit la formation d'anticorps capables de reconnaître des antigènes de souches bactériennes impliquées dans les parodontites.

Les germes sélectionnés pour cette étude sont, d'une part ceux entrant dans la fabrication des constituants de D53, à savoir K. pneumoniae (Kp), S. pneumoniae (Spn), S. pyogenes (Spy), H. influenzae (Hi) et les souches bactériennes les plus fréquemment retrouvées dans différents types de parodontites telles qu'en particulier A. actinomycetemcomitans (Aa) pour la parodontite juvénile, P. gingivalis (Pg) pour les parodontites de l'adulte et F. nucleatum (Fn) pour toutes parodontites.

Après immunisation des souris suivant le protocole indiqué à l'exemple 1, leur sérum prélevé 21 jours après immunisation est testé en ELISA soit sur bactéries entières (premier ELISA), soit sur lysats bactériens (second ELISA).

### Résultats

Les résultats illustrés par la figure unique montrent que les anticorps générés chez la souris immunisée par une composition de type D53 sont capables de reconnaître la plupart des souches parodontales testées qu'elles soient sous forme de bactéries entières ou de lysats bactériens. On peut noter cependant que, globalement, les réponses sont plus intenses sur bactéries entières que sur lysats bactériens.

La plus forte réactivité est notée pour A. actinomycetemcomitans (Aa). Cette forte réactivité pourrait s'expliquer par le fait que :
- phylogénétiquement : Aa était anciennement classé parmi les *Haemophilus*,
- antigéniquement : la membrane externe d'Aa renferme une protéine de membrane externe ou OmpA (pour "outer membrane protein") de 29 kDa ayant une homologie de séquence N-terminale avec l'OmpA d'E. coli (Wilson ME, Hamilton RG, Infect Immun 1991, 59(7): 2505-2512). Or, l'OmpA d'E.coli est elle-même très homologue à la protéine Ompa de K. pneumoniae.

On peut également noter que comme attendu, le sérum anti-J022X réagit fortement sur les souches bactériennes de D53, avec toutefois une plus faible réactivité pour S. pyogenes.

Ces résultats montrent que, chez la souris, l'immunisation avec une composition comprenant des protéoglycanes de Klebsiella pneumoniae, et des ribosomes ou des ARNs ribosomaux de Klebsiella pneumoniae, de Streptococcus pneumoniae, de Streptococcus pyogenes et d'Haemophilus influenzae, telle que le D53, génère des anticorps capables de reconnaître des antigènes de souches bactériennes impliquées et d'incidence élevée dans les maladies parodontales.

## Revendications

1. Utilisation d'une composition comprenant des protéoglycanes de Klebsiella pneumoniae, et des ribosomes ou des ARN ribosomaux de Klebsiella pneumoniae, de Streptococcus pneumoniae, de Streptococcus pyogenes et d'Haemophilus influenzae pour la préparation d'un médicament destiné au traitement et/ou à la prévention des maladies parodontales.

2. Utilisation selon la revendication 1, **caractérisée en ce que** ladite composition comprend des protéoglycanes membranaires purifiés de Klebsiella pneumoniae et des ribosomes de Klebsiella pneumoniae, de Streptococcus pneumoniae, de Streptococcus pyogenes groupe A et d'Haemophilus influenzae.

3. Utilisation selon la revendication 1 ou 2, **caractérisée en ce que** lesdits ribosomes de Klebsiella pneumoniae, de Streptococcus pneumoniae, de Streptococcus pyogenes et d'Haemophilus influenzae contiennent plus de 65 %, de préférence 70 % (p/p) d'ARN.

4. Utilisation selon l'une des revendications 1 à 3, **caractérisée en ce que** le rapport en poids entre lesdits protéoglycanes et lesdits ribosomes ou ARNs ribosomaux est compris entre 1,4 et 1,6, de préférence 1,5.

5. Utilisation selon l'une des revendications 1 à 4, **caractérisée en ce que** les ribosomes ou ARNs ribosomaux de Klebsiella pneumoniae, de Streptococcus pneumoniae, de Streptococcus pyogenes et d'Haemophilus influenzae sont contenus dans ladite composition à un taux respectif compris entre 25 % et 45 %, 20% et 40 %, 20 % et 40 %, et 1 % et 10 % en poids par rapport à la quantité totale de ribosomes ou d'ARNs ribosomaux de Klebsiella pneumoniae, de Streptococcus pneumoniae, de Streptococcus pyogenes et d'Haemophilus influenzae.

6. Utilisation d'une composition selon la revendication 5, **caractérisée en ce que** ledit taux respectif de ribosomes ou d'ARNs ribosomaux de Klebsiella pneumoniae, de Streptococcus pneumoniae, de Streptococcus pyogenes et d'Haemophilus influenzae est de 35 %, 30 %, 30 % et 5 %.

7. Utilisation d'une composition selon l'une des revendications 1 à 6, **caractérisée en ce que** ladite composition comprend en outre un excipient pharmaceutiquement acceptable.

8. Utilisation d'une composition selon la revendication 7, **caractérisée en ce que** l'excipient est choisi parmi les glycérides polyglycosylés insaturés, l'eucalyptol, le dichlorodifluorométhane, le polyvidone et le mannitol.

9. Utilisation selon l'une des revendications 1 à 8, **caractérisée en ce que** ledit médicament est administré par voie nasale, orale, parentérale ou topique.

10. Utilisation selon l'une des revendications 1 à 9, **caractérisée en ce que** ledit médicament est présenté sous forme d'une suspension pour inhalation de composition suivante :
pour un flacon pressurisé de 10 ml
- fractions ribosomales :
ribosomes titrés à 70 % d'ARN 2 mg
associés dans les proportions suivantes :
ribosomes de Klebsiella pneumoniae 35 parties
ribosomes de Streptococcus pneumoniae 30 parties
ribosomes de Streptococcus pyogenes groupe A 30 parties
ribosomes d'Haemophilus influenzae 5 parties
- fractions membranaires :
protéoglycanes de Klebsiella pneumoniae 3 mg
- excipients :
glycérides polyglycosylés insaturés, eucalyptol, dichlorodifluorométhane.

11. Utilisation selon l'une des revendications 1 à 9, **caractérisée en ce que** ledit médicament est présenté sous forme d'un sachet de granulé pour solution buvable de composition suivante :
- fractions ribosomales :
ribosomes titrés à 70 % d'ARN 0,750 mg
associés dans les proportions suivantes :
ribosomes de Klebsiella pneumoniae 35 parties
ribosomes de Streptococcus pneumoniae 30 parties
ribosomes de Streptococcus pyogenes groupe A 30 parties
ribosomes d'Haemophilus influenzae 5 parties
- fractions membranaires :
protéoglycanes de Klebsiella pneumoniae 1,125 mg
- excipients :
polyvidone, D-mannitol.

12. Utilisation selon l'une des revendications 1 à 9, **caractérisée en ce que** ledit médicament est présenté sous forme d'une poudre lyophilisée à réhydrater pour usage parentérale de composition suivante :
• lyophilisat : par flacon
- fractions ribosomales :
ribosomes titrés à 70 % d'ARN 0,010 mg
associés dans les proportions suivantes :
ribosomes de Klebsiella pneumoniae 35 parties
ribosomes de Streptococcus pneumoniae 30 parties
ribosomes de Streptococcus pyogenes groupe A 30 parties
ribosomes d'Haemophilus influenzae 5 parties
- fractions membranaires :
protéoglycanes de Klebsiella pneumoniae 0,015 mg
- excipient :
mannitol
• solvant : par ampoule
chlorure de sodium 3,5 mg eau pour préparations injectables 0,5 ml.

13. Utilisation d'une composition comprenant des protéoglycanes de Klebsiella pneumoniae, et des ribosomes ou des ARN ribosomaux de Klebsiella pneumoniae, de Streptococcus pneumoniae, de Streptococcus pyogenes et d'Haemophilus influenzae pour la préparation d'une composition cosmétique destinée au traitement et/ou à la prévention de la plaque dentaire.

## Patentansprüche

1. Verwendung einer Zusammensetzung, umfassend Proteoglycane von Klebsiella pneumoniae und Ribosomen oder ribosomale RNAs von Klebsiella pneumoniae, von Streptococcus pneumoniae, von Streptococcus pyogenes und von Haemophilus influenzae für die Herstellung eines Arzneimittels, das für die Behandlung und/oder die Verhinderung von parodontalen Erkrankungen bestimmt ist.

2. Verwendung nach Anspruch 1, **dadurch gekennzeichnet, dass** die Zusammensetzung gereinigte Membran-Proteoglycane von Klebsiella pneumoniae und Ribosomen von Klebsiella pneumoniae, von Streptococcus pneumoniae, von Streptococcus pyogenes Gruppe A und von Haemophilus influenzae umfasst.

3. Verwendung nach Anspruch 1 oder 2, **dadurch gekennzeichnet, dass** die Ribosomen von Klebsiella pneumoniae, von Streptococcus pneumoniae, von Streptococcus pyogenes und von Haemophilus influenzae mehr als 65%, vorzugsweise 70% (Gew./Gew.) RNA enthalten.

4. Verwendung nach einem der Ansprüche 1 bis 3, **dadurch gekennzeichnet, dass** das Gewichtsverhältnis zwischen den Proteoglycanen und den Ribosomen oder ribosomalen RNAs zwischen 1,4 und 1,6 liegt, vorzugsweise 1,5 ist.

5. Verwendung nach einem der Ansprüche 1 bis 4, **dadurch gekennzeichnet, dass** die Ribosomen oder ribosomalen RNAs von Klebsiella pneumoniae, von Streptococcus pneumoniae, von Streptococcus pyogenes und von Haemophilus influenzae in der Zusammensetzung in einem jeweiligen Anteil zwischen 25 Gew.-% und 45 Gew.-%, 20 Gew.-% und 40 Gew.-%, 20 Gew.-% und 40 Gew.- %, und 1 Gew.-% und 10 Gew.-% bezogen auf die Gesamtmenge von Ribosomen oder ribosomalen RNAs von Klebsiella pneumoniae, von Streptococcus pneumoniae, von Streptococcus pyogenes und von Haemophilus influenzae enthalten sind.

6. Verwendung einer Zusammensetzung nach Anspruch 5, **dadurch gekennzeichnet, dass** der jeweilige Anteil von Ribosomen oder ribosomalen RNAs von Klebsiella pneumoniae, von Streptococcus pneumoniae, von Streptococcus pyogenes und von Haemophilus influenzae 35%, 30%, 30% und 5% beträgt.

7. Verwendung einer Zusammensetzung nach einem der Ansprüche 1 bis 6, **dadurch gekennzeichnet, dass** die Zusammensetzung außerdem einen pharmazeutisch annehmbaren oder verträglichen Träger umfasst.

8. Verwendung einer Zusammensetzung nach Anspruch 7, **dadurch gekennzeichnet, dass** der Träger unter den ungesättigten polyglycosylierten Glyceriden, Eucalyptol, Dichlordifluormethan, Polyvidon und Mannitol ausgewählt ist.

9. Verwendung nach einem der Ansprüche 1 bis 8, **dadurch gekennzeichnet, dass** das Arzneimittel auf nasalem, oralem, parenteralem oder topischem Wege verabreicht wird.

10. Verwendung nach einem der Ansprüche 1 bis 9, **dadurch gekennzeichnet, dass** das Arzneimittel vorliegt in Form einer Suspension für die Inhalation mit der folgenden Zusammensetzung:
für eine unter Druck stehende Flasche von 10 ml:
- ribosomale Anteile:
auf 70% RNA titrierte Ribosomen 2 mg
kombiniert in den folgenden Verhältnissen:
Ribosomen von Klebsiella pneumoniae 35 Teile
Ribosomen von Streptococcus pneumoniae 30 Teile
Ribosomen von Streptococcus pyogenes Gruppe A 30 Teile
Ribosomen von Haemophilus influenzae 5 Teile
- Membrananteile:
Proteoglycane von Klebsiella pneumoniae 3 mg
- Träger:
ungesättigte polyglycosylierte Glyceride, Eucalyptol, Dichlordifluormethan.

11. Verwendung nach einem der Ansprüche 1 bis 9, **dadurch gekennzeichnet, dass** das Arzneimittel vorliegt in Form eines Beutels mit Granulat für eine trinkbare Lösung mit der folgenden Zusammensetzung:
- ribosomale Anteile:
auf 70% RNA titrierte Ribosomen 0,750 mg
kombiniert in den folgenden Verhältnissen:
Ribosomen von Klebsiella pneumoniae 35 Teile
Ribosomen von Streptococcus pneumoniae 30 Teile
Ribosomen von Streptococcus pyogenes Gruppe A 30 Teile
Ribosomen von Haemophilus influenzae 5 Teile
- Membrananteile:
Proteoglycane von Klebsiella pneumoniae 1,125 mg
- Träger:
Polyvidon, D-Mannitol.

12. Verwendung nach einem der Ansprüche 1 bis 9, **dadurch gekennzeichnet, dass** das Arzneimittel vorliegt in Form eines lyophilisierten Pulvers zur Rehydratisierung für eine parenterale Verwendung mit der folgenden Zusammensetzung:
• Lyophilisat: pro Flasche
- ribosomale Anteile:
auf 70% RNA titrierte Ribosomen 0,010 mg
kombiniert in den folgenden Verhältnissen:
Ribosomen von Klebsiella pneumoniae 35 Teile
Ribosomen von Streptococcus pneumoniae 30 Teile
Ribosomen von Streptococcus pyogenes Gruppe A 30 Teile
Ribosomen von Haemophilus influenzae 5 Teile
- Membrananteile:
Proteoglycane von Klebsiella pneumoniae 0,015 mg
- Träger:
Mannitol
• Lösemittel: pro Ampulle
Natriumchlorid 3,5 mg
Wasser für injizierbare Zubereitungen 0,5 ml

13. Verwendung einer Zusammensetzung, umfassend Proteoglycane von Klebsiella pneumoniae und Ribosomen oder ribosomale RNAs von Klebsiella pneumoniae, von Streptococcus pneumoniae, von Streptococcus pyogenes und von Haemophilus influenzae für die Herstellung einer kosmetischen Zusammensetzung, die für die Behandlung und/oder die Verhinderung von Zahnbelag bestimmt ist.

## Claims

1. Use of a composition comprising proteoglycans of klebsiella pneumoniae, and ribosomes or ribosomal RNAs of klebsiella pneumoniae, of streptococcus pneumoniae, of streptococcus pyogenes and of haemophilus influenzae, for preparing a medicine product intended for the treatment and/or the prevention of periodontal diseases.

2. Use according to Claim 1, **characterized in that** said composition comprises purified membrane proteoglycans of klebsiella pneumoniae and ribosomes of klebsiella pneumoniae, of streptococcus pneumoniae, of streptococcus pyogenes group A and of haemophilus influenzae.

3. Use according to Claim 1 or 2, **characterized in that** said ribosomes of klebsiella pneumoniae, of streptococcus pneumoniae, of streptococcus pyogenes and of haemophilus influenzae contain more than 65%, preferably 70% (w/w) of RNA.

4. Use according to one of Claims 1 to 3, **characterized in that** the weight ratio between said proteoglycans and said ribosomes or ribosomal RNAs is between 1.4 and 1.6, preferably 1.5.

5. Use according to one of Claims 1 to 4, **characterized in that** the ribosomes or ribosomal RNAs of klebsiella pneumoniae, of streptococcus pneumoniae, of streptococcus pyogenes and of haemophilus influenzae are contained in said composition at a respective amount of between 25% and 45%, 20% and 40%, 20% and 40%, and 1% and 10% by weight relative to the total amount of ribosomes or ribosomal RNAs of klebsiella pneumoniae, of streptococcus pneumoniae, of streptococcus pyogenes and of haemophilus influenzae.

6. Use of a composition according to Claim 5, **characterized in that** said respective amount of ribosomes or ribosomal RNAs of klebsiella pneumoniae, of streptococcus pneumoniae, of streptococcus pyogenes and of haemophilus influenzae is 35%, 30%, 30% and 5%.

7. Use of a composition according to one of Claims 1 to 6, **characterized in that** said composition also comprises a pharmaceutically acceptable excipient.

8. Use of a composition according to Claim 7, **characterized in that** the excipient is chosen from unsaturated polyglycosylated glycerides, eucalyptol, dichlorodifluoromethane, polyvidone and mannitol.

9. Use according to one of Claims 1 to 8, **characterized in that** said medicinal product is administered nasally, orally, parenterally or topically.

10. Use according to one of Claims 1 to 9, **characterized in that** said medicine or product is in the form of a suspension for inhalation, having the following composition:
for a 10 ml pressurized bottle
- ribosomal fractions:
ribosomes started at 70% of RNA 2 mg
combined in the following proportions:
ribosomes of klebsiella pneumoniae 35 parts
ribosomes of streptococcus pneumoniae 30 parts
ribosomes of streptococcus pyogenes group A 30 parts
ribosomes of haemophilus influenzae 5 parts
- membrane fractions:
proteoglycans of klebsiella pneumoniae 3 mg
- excipients:
unsaturated polyglycosylated glycerides, eucalyptol, dichlorodifluoromethane.

11. Use according to one of Claims 1 to 9, **characterized in that** said medicinal product is in the form of a sachet of granules for drinkable solutions, having the following composition:
- ribosome fractions:
ribosomes started at 70% of RNA 0.750 mg
combined in the following proportions:
ribosomes of klebsiella pneumoniae 35 parts
ribosomes of streptococcus pneumoniae 30 parts
ribosomes of streptococcus pyogenes group A 30 parts
ribosomes of haemophilus influenzae 5 parts
- membrane fractions:
proteoglycans of klebsiella pneumoniae 1.125 mg
- excipients:
polyvidone, D-mannitol.

12. Use according to one of Claims 1 to 9, **characterized in that** said medicinal product is in the form of a lyophilized powder to be rehydrated for parenteral use, having the following composition:
• lyophilisate: per flask
- ribosomal fractions:
ribosomes started at 70% of RNA 0.010 mg
combined in the following proportions:
ribosomes of klebsiella pneumoniae 35 parts
ribosomes of streptococcus pneumoniae 30 parts
ribosomes of streptococcus pyogenes group A 30 parts
ribosomes of haemophilus influenzae 5 parts
- membrane fractions:
proteoglycans of klebsiella pneumoniae 0.015 mg
- excipient:
mannitol
• solvent Per vial
sodium chloride 3.5 mg
water for injectable preparations 0.5 ml.

13. Use of a composition comprising proteoglycans of klebsiella pneumoniae, and ribosomes or ribosomal RNAs of klebsiella pneumoniae, of streptococcus pneumoniae, of streptococcus pyogenes and of haemophilus influenzae, for preparing a cosmetic composition intended for the treatment and/or the prevention of dental plaque.
